# EUROPÄISCHE PATENTSCHRIFT

(11) **EP 2 318 337 B1**
(45) Veröffentlichungstag und Bekanntmachung des Hinweises auf die Patenterteilung: **27.06.2012**
(21) Anmeldenummer: 09780790.3
(22) Anmeldetag: 17.07.2009
(51) Int. Cl.: C07C 7/12

(54) **VERFAHREN ZUR TECHNISCHEN GEWINNUNG VON PROPEN**
METHOD FOR TECHNICAL EXTRACTION OF PROPENE
PROCÉDÉ D'OBTENTION INDUSTRIELLE DU PROPÈNE

(30) Priorität: 21.07.2008 EP 08160796
(43) Veröffentlichungstag der Anmeldung: 11.05.2011
(73) Patentinhaber: BASF SE, 67056 Ludwigshafen (DE)
(72) Erfinder: SCHUBERT, Markus, 67071 Ludwigshafen (DE); RICHTER, Ingo, 68723 Schwetzingen (DE); MÜLLER, Ulrich, 67435 Neustadt (DE); POPLOW, Frank, 51491 Overath (DE); DOLAN, William, Pennsylvania Pennsylvania 19067 (US); KIENER, Christoph, 67256 Weisenheim am Sand (DE)
(86) Internationale Anmeldenummer: PCT/EP2009/059252
(87) Internationale Veröffentlichungsnummer: WO 2010/010050

(56) Entgegenhaltungen:
- WO-A-2007/113085
- WO-A-2007/113118

## Beschreibung

Die vorliegende Erfindung betrifft Verfahren zur technischen Gewinnung von Propen aus einem zumindest Propen und Propan enthaltenden Gasstrom sowie die Verwendung eines porösen metallorganischen Gerüstmaterials zur Propenanreicherung.

Propen stellt ein wichtiges Wertprodukt dar, das beispielsweise als Ausgangsstoff für die Polypropenherstellung dient.

Propen kommt wie dessen homologe Olefine in Erdgas oder Erdöl nur in sehr geringen Mengen vor. Daher ist dessen direkte Gewinnung durch Isolierung aus natürlichen Quellen nicht von wirtschaftlicher Bedeutung.

Es existieren jedoch eine Vielzahl großtechnischer Verfahren, bei denen zunächst eine Mischung insbesondere aus Propan und Propen entsteht, zum Beispiel Propandehydrierung, FCC- oder Steam-Cracken, MTO (Methanol to Olefin) etc. Daher ist bei allen Verfahren die Abtrennung des Propens aus dem Kohlenwasserstof-Produktgemisch erforderlich. Dies trifft auch auf die Gewinnung von Propen durch Olefin-Metathese zu. Gegebenenfalls müssen dieser Trennung mehrere Reinigungsschritte vorangestellt werden.

Eine Möglichkeit der Trennung besteht in einer Destillation, die jedoch insbesondere aufgrund der ähnlichen Siedepunkte von Propan und Propen (nach VDI-Wärmeatlas 5,7 K bei 1 bar; 6,1 K bei 5 bar) und der hohen Reinheitsanforderungen an das Propen eine hohe Anzahl theoretischer Böden benötigt und sich entsprechend aufwendig und teuer gestaltet.

Ein der Destillation überlegenes Verfahren zur Abtrennung und Gewinnung von Propen stellt die Adsorption dar.

Klassische Adsorptionsmittel für die technische Gewinnung von Propen aus Gasgemischen, die neben Propen weitere Kohlenwasserstoffe, insbesondere Propan enthalten, sind Zeolithe.

Solche Zeolith-basierten Verfahren sind von F. A. Da Silva et al., Int. Eng. Chem. Res. 40 (2001), 5758 - 5774; F. A. Da Silva et al., AlChE Journal 47 (2001), 341 - 357 und C.A. Grande et al., Int. Eng. Chem. Res. 44 (2005), 8815 - 8829 beschrieben.

In WO-A 2007/113118 und WO-A 2007/113085 werden ebenfalls ein Verfahren zur technischen Gewinnung von Propen mit Hilfe von metallorganischen Gerüstmaterialien beschrieben, wobei hier jedoch - wie üblich - Propen aus dem Gasstrom durch Adsorption entfernt wird.

Trotz der im Stand der Technik bekannten Verfahren zur technischen Gewinnung von Propen besteht weiterhin ein Bedarf an alternativen Verfahren.

Eine Aufgabe der vorliegenden Erfindung liegt somit darin, solche Verfahren zur Verfügung zu stellen.

Die Aufgabe wird gelöst durch ein Verfahren zur technischen Gewinnung von Propen aus einem zumindest Propen und Propan enthaltenden Gasstrom, den Schritt enthaltend
- In Kontakt bringen des Gasstromes mit einem Adsorbens enthaltend ein poröses metallorganisches Gerüstmaterial enthaltend mindestens eine an mindestens ein Metallion koordinativ gebundene mindestens zweizähnige organische Verbindung, wobei das Adsorbens mit Propan beladen wird und der Gasstrom dadurch einen erhöhten Propenanteil aufweist, wobei die mindestens zweizähnige organische Verbindung ein Imidazolat ist, das unsubstituiert ist oder einen oder mehrere Substituenten unabhängig voneinander ausgewählt aus der Gruppe bestehend aus Halogen, C₁₋₆ Alkyl, Phenyl, NH₂, NH(C₁₋₆ Alkyl), N(C₁₋₆ Alkyl)₂, OH, O-Phenyl und O-C₁₋₆ Alkyl aufweist.

Es hat sich nämlich gezeigt, dass im Gegensatz zu dem üblichen Adsorptionsverhalten, welches von metallorganischen Gerüstmaterialien im Allgemeinen und Zeolithen bekannt ist, das spezielle Imidazolat-basierte poröse metallorganische Gerüstmaterial bevorzugt Propan adsorbiert, so dass ein Gasstrom der neben Propen zumindest Propan enthält, nach in Kontakt bringen mit einem Adsorbens das spezielle metallorganische Gerüstmaterial enthaltend im Hinblick auf Propen angereichert ist. Dies hat den Vorteil, dass das Wertprodukt im Gasstrom verbleibt und somit eine anschließende Desorption nicht erforderlich ist. Eine Desorption ist normalerweise nachteilig, da üblicherweise dabei der Druck abgesenkt werden muss, so dass eine Propen-reiche Fraktion vor der weiteren Verarbeitung meist dekomprimiert werden muss, was einen erheblichen Energieverbrauch mit sich bringt. Zudem ist es bei Trennungen nach dem Druck- und/oder Temperaturwechselprinzip auch oftmals schwierig, die absorbierte Komponente in der Desorptionsphase in hoher Reinheit zu isolieren, da nicht vollständig vermieden werden kann, dass der nicht getrennte Anteil des Zwischenkornvolumens in das Produkt gerät.

Der Anteil des Propens am Gasstrom kann unterschiedliche Werte annehmen, wobei dieser Anteil stark von der Quelle des Gasstroms abhängt. Hierbei ist jedoch insbesondere der Anteil an Propan wichtig, der bezogen auf die Summe der Volumenanteile an Propen und Propan im Gasstrom enthalten ist, da diese Trennung das Hauptproblem darstellt. Die Abtrennung anderer Bestandteile des Gasstroms kann gegebenenfalls in einem vorgelagerten Schritt erfolgen und kann auch von anderen Adsorbentien anstelle eines porösen metallorganischen Gerüstmaterials erfolgen. Weiterhin können hierfür auch andere Methoden, wie die Destillation, eingesetzt werden.

Neben Propen enthält der Gasstrom zumindest Propan. Typischerweise können zusätzlich mehrere weitere Kohlenwasserstoffe enthalten sein.

Es ist bevorzugt, dass der Gasstrom 20 bis 80 Vol-% Propen, bezogen auf die Summe der Volumenanteile an Propen und dem weiteren Kohlenwasserstoff oder den weiteren Kohlenwasserstoffen im Gasstrom enthält.

Mehr bevorzugt beträgt der Anteil an Propen 30 bis 70 Vol-%. Weiterhin ist bevorzugt, dass auch der Absolutgehalt an Propen im Gasstrom diese Werte annehmen kann.

In einer bevorzugten Ausführungsform handelt es sich bei dem Gasstrom um einen gegebenenfalls gereinigten Produktstrom aus der Propenherstellung.

Solche Produktströme enthalten neben Propen typischerweise weitere homologe Alkane, insbesondere Propan, und Alkene sowie weitere gasförmige Bestandteile, die jedoch durch einfache Reinigungsschritte entfernt werden können. Ein Beispiel eines solchen durch Reinigung entfernbaren Bestandteiles eines Produktstroms aus der Propenherstellung stellt gasförmiges Wasser dar, das durch ein gängiges Trocknungsmittel oder durch Kondensation bei Verdichtung und Abkühlen entsprechend entfernt werden kann. Ein weiteres Beispiel stellt Kohlendioxid dar, das durch eine einfache Gaswäsche entfernt werden kann. Weitere Beispiele sind Ethin und Allene, die zuvor selektiv hydriert werden können.

Alternativ zu Crack-Verfahren kann auch eine Olefinumwandlung Olefin-Metathese zur Propenherstellung erfolgen. Hierbei können Ethen und 2-Buten zu Propen umgesetzt werden.

Schließlich stellt auch die Methanol- beziehungsweise Dimethylether-Umwandlung eine mehr oder weniger gezielte Propenherstellung dar. Solche Umsetzungen werden auch als "Methanol to Olefins" bezeichnet. Diese Umsetzungen an Zeolithen (ZSM-5 oder SAPO) zielen entweder auf eine Propen/Ethen-Herstellung neben Spuren an C₅⁺ oder auf eine Propen/Benzin-Herstellung ab. In beiden Fällen muss Propen u.a. von mehr oder weniger großen Mengen an LPG abgetrennt werden.

Eine besonders bevorzugte Propenherstellung zur Erzeugung eines gegebenenfalls gereinigten Produktstroms stellt die Propandehydrierung dar.

Ein bevorzugter Gegenstand der vorliegenden Erfindung liegt somit darin, dass der gegebenenfalls gereinigte Produktstrom aus einem Crack-Verfahren, einer Propandehydrierung, einer Olefin-Metathese oder einer Methanolumwandlung zur Propenherstellung, insbesondere aus einer Propandehydrierung stammt.

Es ist ebenfalls möglich, dass entsprechende Mischungen solcher unterschiedlicher Produktströme eingesetzt werden.

Ein bevorzugtes Verfahren zur Propandehydrierung enthält die Schritte:
A) ein Propan enthaltender Einsatzgasstrom a wird bereitgestellt;
B) der Propan enthaltende Einsatzgasstrom a, gegebenenfalls Wasserdampf und gegebenenfalls ein sauerstoffhaltiger Gasstrom werden in eine Dehydrierzone eingespeist und Propan wird einer Dehydrierung zu Propen unterworfen, wobei ein Produktgasstrom b enthaltend Propan, Propen, Methan, Ethan, Ethen, Wasserstoff, gegebenenfalls Kohlenmonoxid, Kohlendioxid, Wasserdampf und Sauerstoff erhalten wird;
C) der Produktgasstrom b wird abgekühlt, gegebenenfalls verdichtet und es wird Wasserdampf durch Kondensation abgetrennt, wobei ein an Wasserdampf abgereicherter Produktgasstrom c erhalten wird.

In einem ersten Verfahrensteil A) wird ein Propan enthaltender Einsatzgasstrom a bereitgestellt. Dieser enthält im Allgemeinen mindestens 80 Vol-% Propan, vorzugsweise 90 Vol-% Propan. Daneben enthält der propanhaltige Einsatzgasstrom a im Allgemeinen noch Butane (n-Butan, iso-Butan). Typische Zusammensetzungen des propanhaltigen Einsatzgasstroms sind in DE-A 102 46 119 und DE-A 102 45 585 offenbart. Üblicherweise wird der propanhaltige Einsatzgasstrom a aus liquid petroleum gas (LPG) gewonnen.

In einem Verfahrensteil B) wird der Propan enthaltende Einsatzgasstrom in eine Dehydrierzone eingespeist und einer im Allgemeinen katalytischen Dehydrierung unterworfen. Dabei wird Propan in einem Dehydrierreaktor an einem dehydrieraktiven Katalysator teilweise zu Propen dehydriert. Daneben fallen Wasserstoff und in geringen Mengen Methan, Ethan, Ethen und C₄⁺-Kohlenwasserstoffe (n-Butan, iso-Butan, Butene, Butadien) an. Außerdem fallen im Allgemeinen Kohlenstoffoxide (CO, CO₂), insbesondere CO₂, Wasserdampf und gegebenenfalls in geringem Umfang Inertgase im Produktgasgemisch der katalytischen Propan-Dehydrierung an. Der Produktgasstrom der Dehydrierung enthält im Allgemeinen Wasserdampf, der bereits dem Dehydriergasgemisch zugesetzt und/oder - bei Dehydrierung in Gegenwart von Sauerstoff (oxidativ oder nicht-oxidativ) - bei der Dehydrierung gebildet wird. Inertgase (Stickstoff) werden bei Durchführung der Dehydrierung in Gegenwart von Sauerstoff mit dem eingespeisten sauerstoffhaltigen Gasstrom in die Dehydrierzone eingebracht, sofern kein reiner Sauerstoff eingespeist wird. Wird ein sauerstoffhaltiges Gas eingespeist, so liegt dessen Sauerstoff-Gehalt im Allgemeinen bei mindestens 40 Vol-%, bevorzugt mindestens 80 Vol-%, besonders bevorzugt mindestens 90 Vol-%, insbesondere technisch reiner Sauerstoff mit einem Sauerstoffgehalt > 99%, um einen zu hohen Inertgasanteil im Produktgasgemisch zu vermeiden. Daneben liegt im Produktgasgemisch nicht umgesetztes Propan vor.

Die Propan-Dehydrierung kann grundsätzlich in allen aus dem Stand der Technik bekannten Reaktortypen durchgeführt werden. Eine vergleichsweise ausführliche Beschreibung von erfindungsgemäß geeigneten Reaktortypen enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Die Dehydrierung kann als oxidative oder nicht-oxidative Dehydrierung durchgeführt werden. Die Dehydrierung kann isotherm oder adiabat durchgeführt werden. Die Dehydrierung kann katalytisch im Festbett-, Wanderbett- oder Wirbelbettreaktor durchgeführt werden.

Die oxidative katalytische Propan-Dehydrierung wird bevorzugt autotherm durchgeführt. Dazu wird dem Reaktionsgasgemisch der Propan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Ein Merkmal der nicht-oxidativen Fahrweise gegenüber einer oxidativen Fahrweise ist die zumindest intermediäre Bildung von Wasserstoff, die sich im Vorhandensein von Wasserstoff im Produktgas der Dehydrierung niederschlägt. Bei der oxidativen Dehydrierung findet sich kein freier Wasserstoff im Produktgas der Dehydrierung.

Eine geeignete Reaktorform ist der Festbettrohr- oder Rohrbündelreaktor. Bei diesen befindet sich der Katalysator (Dehydrierungskatalysator und gegebenenfalls spezieller Oxidationskatalysator) als Festbett in einem Reaktionsrohr oder in einem Bündel von Reaktionsrohren. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung, aber auch zwischen 3 und 8 bar bei Verwendung einer hohen Wasserdampfverdünnung (entsprechend dem so genannten "steam active reforming process" (STAR-Prozess) oder dem Linde-Verfahren) zur Dehydrierung von Propan oder Butan von Phillips Petroleum Co. Typische Katalysatorbelastungen (GHSV) liegen bei 500 bis 2000 h-¹, bezogen auf eingesetzten Kohlenwasserstoff. Die Katalysatorgeometrie kann beispielsweise kugelförmig oder zylindrisch (hohl oder voll) sein.

Die katalytische Propan-Dehydrierung kann auch, entsprechend dem Snamprogetti/Yarsintez-FBD-Prozess, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet.

Der Arbeitsdruck beträgt typischerweise 1 bis 2 bar, die Dehydriertemperatur in der Regel 550 bis 600°C. Die für die Dehydrierung erforderliche Wärme kann dabei in das Reaktionssystem eingebracht werden, indem der Dehydrierkatalysator auf die Reaktionstemperatur vorerhitzt wird. Durch die Zumischung eines Sauerstoff enthaltenden Co-Feeds kann teilweise auf die Vorerhitzer verzichtet werden, und die benötigte Wärme wird direkt im Reaktorsystem durch Verbrennung von Wasserstoff und/oder Kohlenwasserstoffen in Gegenwart von Sauerstoff erzeugt. Gegebenenfalls kann zusätzlich ein Wasserstoff enthaltender Co-Feed zugemischt werden.

Die katalytische Propan-Dehydrierung kann in einem Hordenreaktor durchgeführt werden. Wird die Dehydrierung unter Einspeisung eines sauerstoffhaltigen Gasstroms autotherm durchgeführt, so wird sie bevorzugt in einem Hordenreaktor durchgeführt. Dieser enthält ein oder mehrere aufeinander folgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer Ausführungsform. In einer weiteren bevorzugten Ausführungsform wird die Dehydrierung in einem Hordenreaktor mit 3 Katalysatorbetten durchgeführt.

Im Allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des Propans benötigte Wärmemenge erzeugt wird. Im Allgemeinen beträgt die insgesamt zugeführte Sauerstoffmenge, bezogen auf die Gesamtmenge des Propans, 0,001 bis 0,8 mol/mol, bevorzugt 0,001 bis 0,6 mol/mol, besonders bevorzugt 0,02 bis 0,5 mol/mol. Sauerstoff kann entweder als reiner Sauerstoff oder als sauerstoffhaltiges Gas, welches Inertgase enthält, eingesetzt werden. Um hohe Propan- und Propen-Verluste bei der Aufarbeitung (siehe unten) zu vermeiden ist es jedoch wesentlich, dass der Sauerstoff-Gehalt des eingesetzten sauerstoffhaltigen Gases hoch ist und mindestens 40 Vol-%, bevorzugt mindestens 80 Vol-%, besonders bevorzugt mindestens 90 Vol% beträgt. Besonders bevorzugtes sauerstoffhaltiges Gas ist technisch reiner Sauerstoff mit einem O₂-Gehalt von ca. 99 Vol-%.

Der zur Wärmeerzeugung verbrannte Wasserstoff ist der bei der katalytischen Propan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stellen des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im Allgemeinen 400 bis 1100 °C, der Druck im letzten Katalysatorbett des Hordenreaktors im Allgemeinen 0,2 bis 15 bar, bevorzugt 1 bis 10 bar, besonders bevorzugt 1 bis 5 bar. Die Belastung (GHSV) beträgt im Allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliziumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Geeignete Katalysatorformkörper-Geometrien sind Stränge, Sterne, Ringe, Sattel, Kugeln, Schäume und Monolithen mit charakteristischen Abmessungen von 1 bis 100 mm.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen Propan-Dehydrierung sind die Katalysatoren gemäß den Beispielen 1, 2, 3 und 4 der DE-A 199 37 107.

Die autotherme Propandehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt. Durch die Verdünnung mit Wasserdampf wird der Gleichgewichtsumsatzgrad erhöht.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Gegebenenfalls wird der Katalysator nach der Regenerierung mit einem wasserstoffhaltigen Gas reduziert.

Der Produktgasstrom b kann in zwei Teilströme aufgetrennt werden, wobei ein Teilstrom in die autotherme Dehydrierung zurückgeführt wird, entsprechend der in DE-A 102 11 275 und DE-A 100 28 582 beschriebenen Kreisgasfahrweise.

Die Propan-Dehydrierung kann als oxidative Dehydrierung durchgeführt werden. Die oxidative Propan-Dehydrierung kann als homogene oxidative Dehydrierung oder als heterogen katalysierte oxidative Dehydrierung durchgeführt werden.

Gestaltet man im Rahmen des erfindungsgemäßen Verfahrens die Propan-Dehydrierung als eine homogene Oxidehydrierung, so lässt sich diese prinzipiell wie in den Schriften US-A 3,798,283, CN-A 1,105,352, Applied Catalysis, 70 (2), 1991, S. 175 bis 187, Catalysis Today 13, 1992, S. 673 bis 678 und der älteren Anmeldung DE-A 1 96 22 331 beschrieben durchführen.

Die Temperatur der homogenen Oxidehydrierung beträgt im Allgemeinen von 300 bis 700 °C, vorzugsweise von 400 bis 600 °C, besonders bevorzugt von 400 bis 500 °C. Der Druck kann 0,5 bis 100 bar oder 1 bis 50 bar betragen. Häufig wird er bei 1 bis 20 bar, insbesondere bei 1 bis 10 bar liegen.

Die Verweilzeit des Reaktionsgasgemisches unter Oxidehydrierbedingungen liegt üblicherweise bei 0,1 bzw. 0,5 bis 20 sec, vorzugsweise bei 0,1 bzw. 0,5 bis 5 sec. Als Reaktor kann z.B. ein Rohrofen oder ein Rohrbündelreaktor verwendet werden, wie z.B. ein Gegenstromrohrofen mit Rauchgas als Wärmeträger, oder ein Rohrbündelreaktor mit Salzschmelze als Wärmeträger.

Das Propan zu Sauerstoff-Verhältnis im einzusetzenden Ausgangsgemisch kann 0,5 : 1 bis 40 : 1 betragen. Vorzugsweise beträgt das Molverhältnis von Propan zu molekularem Sauerstoff im Ausgangsgemisch ≤ 6 : 1, bevorzugt ≤ 5 : 1 beträgt. In der Regel wird vorgenanntes Verhältnis ≥ 1 : 1 , beispielsweise ≥ 2 : 1 betragen. Das Ausgangsgemisch kann weitere, im Wesentlichen inerte Bestandteile wie H₂O, CO₂, CO, N₂, Edelgase und/oder Propen umfassen. Propen kann in der aus der Raffinerie kommenden C₃-Fraktion enthalten sein. Günstig für eine homogene oxidative Dehydrierung von Propan zu Propen ist es, wenn das Verhältnis der Oberfläche des Reaktionsraumes zum Volumen des Reaktionsraumes möglichst klein ist, da die homogene oxidative Propan-Dehydrierung nach einem radikalischen Mechanismus abläuft und die Reaktionsraumoberfläche in der Regel als Radikalfänger wirkt. Besonders günstige Oberflächenmaterialien sind Aluminiumoxide, Quarzglas, Borosilikate, Edelstahl und Aluminium.

Gestaltet man im Rahmen des erfindungsgemäßen Verfahrens die erste Reaktionsstufe als eine heterogen katalysierte Oxidehydrierung, so lässt sich diese prinzipiell wie in den Schriften US-A 4,788,371, CN-A 1,073,893, Catalysis Letters 23 (1994) 103-106, W. Zhang, Gaodeng Xuexiao Huaxue Xuebao, 14(1993)566, Z. Huang, Shiyou Huagong, 21(1992)592, WO 97/36849, DE-A 1 97 53 817, US-A 3,862,256, US-A 3,887,631, DE-A 1 95 30 454, US-A 4,341,664, J. of Catalysis 167, 560 - 569 (1997), J. of Catalysis 167, 550 - 559 (1997), Topics in Catalysis 3 (1996) 265 - 275, US-A 5,086,032, Catalysis Letters 10 (1991) 181 - 192, Ind. Eng. Chem. Res. 1996, 35, 14 - 18, US-A 4,255,284, Applied Catalysis A: General, 100 (1993) 111 - 130, J. of Catalysis 148, 56 - 67 (1994), V. Cortés Corberän and S. Vic Bellón (Editors), New Developments in Selective Oxidation II, 1994, Elsevier Science B.V., S. 305 - 313, 3rd World Congress on Oxidation Catalysis R.K. Grasselli, S.T. Oyama, A.M. Gaffney and J.E. Lyons (Editors), 1997, Elsevier Science B.V., S. 375 ff beschrieben durchführen. Insbesondere können alle in den vorgenannten Schriften genannten Oxidehydrierkatalysatoren eingesetzt werden. Das für die vorgenannten Schriften gesagte gilt auch für:
i) Otsuka, K.; Uragami, Y.; Komatsu, T.; Hatano, M. in Natural Gas Conversion, Stud. Surf. Sci. Catal.; Holmen A.; Jens, K.-J.; Kolboe, S., Eds.; Elsevier Science: Amsterdam, 1991; Vol. 61, p 15;
ii) Seshan, K.; Swaan, H.M.; Smits, R.H.H.; van Ommen, J.G.; Ross, J.R.H. in New Developments in Selective Oxidation; Stud. Surf. Sci. Catal.; Centi, G.; Trifirö, F., Eds.; Elsevier Science: Amsterdam 1990; Vol. 55, p 505;
iii) Smits, R.H.H.; Seshan, K.; Ross, J.R.H. in New Developments in Selective Oxidation by Heterogeneous Catalysis; Stud. Surf. Sci. Catal.; Ruiz, P.; Delmon, B., Eds.; EIsevier Science: Amsterdam, 1992 a; Vol. 72, p 221;
iv) Smits, R.H.H.; Seshan, K.; Ross, J.R.H. Proceedings, Symposium on Catalytic Selective Oxidation, Washington DC; American Chemical Society: Washington, DC, 1992 b; 1121;
v) Mazzocchia, C.; Aboumrad, C.; Daigne, C.; Tempesti, E.; Herrmann, J.M.; Thomas, G. Catal. Lett. 1991, 10, 181;
vi) Bellusi, G.; Conti, G.; Perathonar, S.; Trifirö, F. Proceedings, Symposium on Catalytic Selective Oxidation, Washington, DC; American Chemical Society: Washington, DC, 1992; p 1242;
vii) Ind. Eng. Chem. Res. 1996, 35, 2137 - 2143 und
viii) Symposium on Heterogeneons Hydrocarbon Oxidation Presented before the Division of Petroleum Chemistry, Inc. 211 th National Meeting, American Chemical Society New Orleans, LA, March 24 - 29, 1996.

Besonders geeignete Oxidehydrierkatalysatoren sind die Multimetalloxidmassen beziehungsweise -katalysatoren A der DE-A 1 97 53 817, wobei die als bevorzugt genannten Multimetalloxidmassen bzw. -katalysatoren A ganz besonders günstig sind. D.h., als Aktivmassen kommen insbesondere Multimetalloxidmassen der allgemeinen Formel I

M¹ₐMo_{1-b}M²_{b}Oₓ (I),

mit
- M¹ =: Co, Ni, Mg, Zn, Mn und/oder Cu,
- M² =: W, V, Te, Nb, P, Cr, Fe, Sb, Ce, Sn und/oder La,
- a =: 0,5 bis 1,5,
- b =: 0 bis 0,5 sowie
- x =: eine Zahl, die durch die Wertigkeit und Häufigkeit der von Sauerstoff verschiedenen Elemente in I bestimmt wird,
in Betracht.

Weitere als Oxidehydrierungskatalysatoren geeignete Multimetalloxidmassen sind nachstehend genannt:
Geeignete Mo-V-Te/Sb-Nb-O-Multimetalloxidkatalysatoren sind in EP-A 0 318 295, EP-A 0 529 853, EP-A 0 603 838, EP-A 0 608 836, EP-A 0 608 838, EP-A 0 895 809, EP-A 0 962 253, EP-A 1 192 987, DE-A 198 35 247, DE-A 100 51 419 und DE-A 101 19 933 offenbart.
Geeignete Mo-V-Nb-O-Multimetalloxidkatalysatoren sind unter anderem beschrieben in E. M. Thorsteinson, T. P. Wilson, F. G. Young, P. H. Kasei, Journal of Catalysis 52 (1978), Seiten 116 bis 132 sowie in US 4,250,346 und EP-A 0 294 845.
Geeignete Ni-X-O-Multimetalloxidkatalysatoren, mit X = Ti, Ta, Nb, Co, Hf, W, Y, Zn, Zr, Al, sind in WO 00/48971 beschrieben.

Prinzipiell können geeignete Aktivmassen in einfacher Weise dadurch hergestellt werden, dass man von geeigneten Quellen ihrer Komponenten ein möglichst inniges, vorzugsweise feinteiliges, ihrer Stöchiometrie entsprechend zusammengesetztes Trockengemisch erzeugt und dieses bei Temperaturen von 450 bis 1000 °C calciniert. Die Calcinierung kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, Sauerstoff und NH₃, CO und/oder H₂) erfolgen. Als Quellen für die Komponenten der Multimetalloxidaktivmassen kommen Oxide und/oder solche Verbindungen in Frage, die durch Erhitzen, wenigstens in Anwesenheit von Sauerstoff, in Oxide überführbar sind. Neben den Oxiden kommen als solche Ausgangsverbindungen vor allem Halogenide, Nitrate, Formiate, Oxalate, Citrate, Acetate, Carbonate, Aminkomplexsalze, Ammoniumsalze und/oder Hydroxide in Betracht.

Die Multimetalloxidmassen können für das erfindungsgemäße Verfahren sowohl in Pulverform als auch zu bestimmten Katalysatorgeometrien geformt eingesetzt werden, wobei die Formgebung vor oder nach der abschließenden Calcinierung erfolgen kann. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Geeignete Hohlzylindergeometrien sind z.B. 7 mm x 7 mm x 4 mm oder 5 mm x 3 mm x 2 mm oder 5 mm x 2 mm x 2 mm (jeweils Länge x Außendurchmesser x Innendurchmesser). Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Schichtdicke der auf den Trägerkörper aufgebrachten Pulvermasse wird zweckmäßigerweise im Bereich 50 bis 500 mm, bevorzugt im Bereich 150 bis 250 mm liegend, gewählt. Als Trägermaterialien können dabei übliche poröse oder unporöse Aluminiumoxide, Siliciumdioxid, Thoriumdioxid, Zirkondioxid, Siliciumcarbid oder Silicate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln, Hohlzylinder oder Sättel mit Abmessungen im Bereich von 1 bis 100 mm bevorzugt werden. Geeignet ist die Verwendung von im Wesentlichen unporösen, oberflächenrauhen, kugelförmigen Trägern aus Steatit, deren Durchmesser 1 bis 8 mm, bevorzugt 4 bis 5 mm beträgt.

Die Reaktionstemperatur der heterogen katalysierten Oxidehydrierung des Propans beträgt im Allgemeinen von 300 bis 600 °C, üblicher Weise von 350 bis 500 °C. Der Druck beträgt 0,2 bis 15 bar, bevorzugt 1 bis 10 bar, beispielsweise 1 bis 5 bar. Drücke oberhalb von 1 bar, z.B. 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie z.B. in der EP-A 700 893 und in der EP-A 700 714 sowie der in diesen Schriften zitierten Literatur beschrieben sind. Die mittlere Verweilzeit des Reaktionsgasgemisches in der Katalysatorschüttung liegt im Normalfall bei 0,5 bis 20 sec. Das Propan zu Sauerstoff Verhältnis im für die heterogen katalysierte Propan-Oxidehydrierung einzusetzenden Reaktionsgasausgangsgemisch kann erfindungsgemäß 0,5: 1 bis 40 : 1 betragen. Von Vorteil ist es, wenn das Molverhältnis von Propan zu molekularem Sauerstoff im Ausgangsgasgemisch ≤ 6 : 1 , vorzugsweise ≤ 5 : 1 beträgt. In der Regel wird vorgenanntes Verhältnis ≥ 1:1, beispielsweise 2 : 1 betragen. Das Ausgangsgasgemisch kann weitere, im Wesentlichen inerte Bestandteile wie H₂O, CO₂, CO, N₂, Edelgase und/oder Propen umfassen. Daneben können in gewissem Umfang noch C₁-, C₂- und C₄-Kohlenwasserstoffe enthalten sein.

Der Produktgasstrom b steht beim Verlassen der Dehydrierzone im Allgemeinen unter einem Druck von 0,2 bis 15 bar, vorzugsweise 1 bis 10 bar, besonders bevorzugt 1 bis 5 bar, und weist eine Temperatur im Bereich von 300 bis 700 °C auf.

Bei der Propan-Dehydrierung wird ein Gasgemisch erhalten, welches im Allgemeinen folgende Zusammensetzung aufweist: 10 bis 80 Vol-% Propan, 5 bis 50 Vol-% Propen, 0 bis 20 Vol-% Methan, Ethan, Ethen und C₄⁺-Kohlenwasserstoffe, 0 bis 30 Vol-% Kohlenstoffoxide, 0 bis 70 Vol-% Wasserdampf und 0 bis 25 Vol-% Wasserstoff sowie 0 bis 50 Vol-% Inertgase.

Bei der bevorzugten autothermen Propan-Dehydrierung wird ein Gasgemisch erhalten, welches im Allgemeinen folgende Zusammensetzung aufweist: 10 bis 80 Vol-% Propan, 5 bis 50 Vol-% Propen, 0 bis 20 Vol-% Methan, Ethan, Ethen und C₄⁺-Kohlenwasserstoffe, 0,1 bis 30 Vol-% Kohlenstoffoxide, 1 bis 70 Vol-% Wasserdampf und 0,1 bis 25 Vol-% Wasserstoff sowie 0 bis 30 Vol-% Inertgase.

In Verfahrensteil C) wird zunächst Wasser aus dem Produktgasstrom b abgetrennt. Die Abtrennung von Wasser wird durch Kondensation durch Abkühlen und ggf. Verdichten des Produktgasstroms b durchgeführt und kann in einer oder mehreren Abkühlungs- und gegebenenfalls Verdichtungsstufen durchgeführt werden. Im Allgemeinen wird der Produktgasstrom b hierzu auf eine Temperatur im Bereich von 20 bis 80 °C, vorzugsweise 40 bis 65 °C abgekühlt. Zusätzlich kann der Produktgasstrom verdichtet werden, im Allgemeinen auf einen Druck im Bereich von 2 bis 40 bar, bevorzugt 5 bis 20 bar, besonders bevorzugt 10 bis 20 bar.

In einer Ausführungsform des erfindungsgemäßen Verfahrens wird der Produktgasstrom b durch eine Kaskade von Wärmetauschern geleitet und so zunächst auf eine Temperatur im Bereich von 50 bis 200 °C abgekühlt und anschließend in einem Quenchturm mit Wasser auf eine Temperatur von 40 bis 80 °C, beispielsweise 55 °C weiter abgekühlt. Dabei kondensiert der größte Teil des Wasserdampfs aus, aber auch ein Teil der im Produktgasstrom b enthaltenen C₄⁺-Kohlenwasserstoffe, insbesondere die C₅⁺-Kohlenwasserstoffe. Geeignete Wärmetauscher sind beispielsweise Direktwärmetauscher und Gegenstrom-Wärmetauscher, wie Gas-Gas-Gegenstrom-Wärmetauscher, und Luftkühler.

Es wird ein von Wasserdampf abgereicherter Produktgasstrom c erhalten. Dieser enthält im Allgemeinen noch 0 bis 10 Vol-% Wasserdampf. Zur praktisch vollständigen Entfernung von Wasser aus dem Produktgasstrom c kann bei Verwendung bestimmter Adsorbentien in Schritt D) eine Trocknung mittels Molekularsieb, insbesondere Molsieb 3A, 4A, 13X oder Aluminiumoxide bevorzugt, oder Membranen vorgesehen werden.

Vor der Durchführung von Schritt (a) des erfindungsgemäßen Verfahrens zur technischen Gewinnung von Propen kann Kohlendioxid durch Gaswäsche oder durch Adsorption an festen Adsorptionsmitteln aus dem Gasstrom c abgetrennt werden. Der Kohlendioxid-Gaswäsche kann eine gesonderte Verbrennungsstufe vorgeschaltet werden, in der Kohlenmonoxid selektiv zu Kohlendioxid oxidiert wird.

Zur CO₂-Abtrennung wird im Allgemeinen Natronlauge, Kalilauge oder eine Alkanolamin-Lösung als Waschflüssigkeit eingesetzt, bevorzugt wird eine aktivierte N-Methyldiethanolamin-Lösung eingesetzt. Im Allgemeinen wird vor Durchführung der Gaswäsche der Produktgasstrom c durch ein- oder mehrstufige Verdichtung auf einen Druck im Bereich von 5 bis 25 bar verdichtet. Es kann ein an Kohlendioxid abgereicherter Strom c mit einem CO₂-Gehalt von im Allgemeinen < 1000 ppm, vorzugsweise < 100 ppm, besonders bevorzugt < 20 ppm erhalten werden.

Bevorzugt ist es jedoch, CO₂ durch Sorption an geeigneten festen Sorptionsmitteln, beispielsweise Molsieb 13X, Calciumoxid, Bariumoxid, Magnesiumoxid oder Hydrotalciten, abzutrennen.

In einer besonders bevorzugten Ausführungsform stellt der so erhaltene gereinigte Produktstrom aus der Propenherstellung den bei dem erfindungsgemäßen Verfahren zur technischen Gewinnung von Propen eingesetzten zumindest Propan und Propen enthaltenden Gasstrom dar.

Das Adsorbens enthält ein poröses metallorganisches Gerüstmaterial enthaltend mindestens eine an mindestens ein Metallion koordinativ gebundene, mindestens zweizähnige organische Verbindung, wobei die mindestens zweizähnige organische Verbindung ein Imidazolat ist, das unsubstituiert ist oder einen oder mehrere Substituenten unabhängig voneinander ausgewählt aus der Gruppe bestehend aus Halogen, C₁₋₆ Alkyl, Phenyl, NH₂, NH(C₁₋₆A1kyl), N(C₁₋₆ Alkyl)₂, OH, O-Phenyl und O-C₁₋₆ Alkyl aufweist.

Hierbei bezeichnet der Begriff "C₁₋₆-Alkyl" einen gesättigten aliphatischen acyclischen Kohlenwasserstoffrest, der verzweigt oder unverzweigt sein kann und 1 bis 6 Kohlenstoffatome aufweist. Beispiele hierfür sind Methyl, Ethyl, n-Propyl, i-Propyl, n-Butyl, i-Butyl, t-Butyl, n-Pentyl, i-Pentyl, n-Hexyl, i-Hexyl.

Das metallorganische Gerüstmaterial, welches in dem erfindungsgemäßen Verfahren zum Einsatz kommt, ist aus dem Stand der Technik bekannt oder kann anhand bekannter Methoden hergestellt werden. Dabei kann das Gerüstmaterial durch nasschemische Fällung aus herkömmlichen Salzen, Alkoholaten oder metallorganischen Vorstufen oder auf elektrochemischem Wege hergestellt werden. Dabei wird das dem mindestens einen Metallion entsprechende Metall als Anodenmaterial zur Verfügung gestellt. Verfahren zur elektrochemischen Herstellung solcher metallorganischer Gerüstmaterialien sind beispielsweise in WO-A 2007/131955 beschrieben.

Das metallorganische Gerüstmaterial gemäß der vorliegenden Erfindung enthält Poren, insbesondere Mirko- und/oder Mesoporen. Mikroporen sind definiert als solche mit einem Durchmesser von 2 nm oder kleiner und Mesoporen sind definiert durch einen Durchmesser im Bereich von 2 bis 50 nm, jeweils entsprechend nach der Definition, wie sie Pure Applied Chem. 57, (1985), 603 - 619, insbesondere auf Seite 606 angegeben ist. Die Anwesenheit von Mikro- und/oder Mesoporen kann mit Hilfe von Sorptionsmessungen überprüft werden, wobei diese Messungen die Aufnahmekapazität der MOF für Stickstoff bei 77 Kelvin gemäß DIN 66131 und/oder DIN 66134 bestimmt.

Vorzugsweise beträgt die spezifische Oberfläche - berechnet nach dem Langmuir-Modell gemäß DIN 66135 (DIN 66131, 66134) für ein Gerüstmaterial in Pulverform bei mehr als 300 m²/g, mehr bevorzugt mehr als 500 m²/g, weiter mehr bevorzugt mehr als 600 m²/g, weiter mehr bevorzugt mehr als 1000 m²/g und besonders bevorzugt mehr als 1500 m²/g.

Als Formkörper weist das erfindungsgemäße metallorganische Gerüstmaterial vorzugsweise eine spezifische Oberfläche von mindestens 50 m²/g, mehr bevorzugt von mindestens 100 m²/g, weiter mehr bevorzugt von mindestens 300 m²/h, weiter mehr bevorzugt von mindestens 750 m²/g und insbesondere von mindestens 1000 m²/g auf.

Das Metall oder die Metalle sind Elemente der Gruppen 2 bis 15 des Periodensystems der Elemente. Im Rahmen der vorliegenden Erfindung sind bevorzugte Metallionen ausgewählt aus der Gruppe der Metalle bestehend aus Kupfer, Eisen, Aluminium, Zink, Magnesium, Zirkonium, Titan, Vanadium, Molybdän, Wolfram, Indium, Calcium, Strontium, Kobalt, Nickel, Platin, Rhodium, Ruthenium, Palladium, Scandium, Yttrium, ein Lanthanid, Mangan und Rhenium. Weiter mehr bevorzugt sind Eisen, Kupfer, Zink, Nickel und Kobalt. Besonders bevorzugt ist Zink.

Ein Lanthanid umfasst La, Ce, Pr, Nd, Pm, Sm, Eu, Gd, Tb, Dy, Ho, Er, Yb und Lu.

Als Metallionen, die über anodische Oxidation im Reaktionsmedium bereit gestellt werden, sind insbesondere Cu²⁺, Cu⁺, Ni²⁺, Ni⁺, Fe³⁺, Fe²⁺, Co³⁺, Co²⁺, Zn²⁺, Mn³⁺, Mn²⁺, Al³⁺, Mg²⁺, Sc³⁺, Y³⁺, Ln³⁺, Re³⁺, V³⁺, In³⁺, Ca²⁺, Sr2⁺, Pt²⁺, TiO²⁺, Ti⁴⁺, ZrO²⁺, Zr⁴⁺, Ru³⁺, Ru²⁺, Mo³⁺, W³⁺, Rh²⁺, Rh⁺, Pd²⁺ und Pd⁺ zu nennen. Besonders bevorzugt sind Zn²⁺, Cu²⁺, Cu⁺, Fe²⁺, Fe³⁺, Ni²⁺, Ni⁺, Co³⁺ und Co²⁺. Insbesondere bevorzugt ist Zn²⁺.

Beispiele für ganz besonders geeignete metallorganische Gerüstmaterialien gemäß der vorliegenden Erfindung sind Zn-2-methylimidazolat und Zn-2-ethylimidazolat. Insbesondere ist Zn-2-methylimidazolat geeignet.

Die metallorganischen Gerüstmaterialien werden im Allgemeinen als Formkörper eingesetzt, beispielsweise als regellose Schüttungen aus Kugeln, Ringen, Stränglingen oder Tabletten oder als geordnete Einbauten wie Packungen, Wabenkörper und Monolithe.

Die Herstellung von Formkörpern ist beispielsweise in WO-A 03/102 000 beschrieben. Bevorzugt ist der Einsatz von Formkörperschüttungen, die möglichst dicht gepackt sitzen. Daher weisen die Formkörper an ihrer schmalsten Stelle bevorzugt maximal 3 mm, mehr bevorzugt maximal 2 mm, ganz besonders bevorzugt maximal 1,5 mm Durchmesser auf. Ganz besonders bevorzugt sind Formkörper in Tablettenform. Alternativ ist ein Einbau in Form einer monolithischen Struktur, da hier die großen Kanäle ebenfalls gut zu spülen sind, während das Material in den Wänden ebenfalls sehr dicht gepackt sitzt.

Typischerweise befindet sich das Adsorbens in einem Adsorber. Neben dem erfindungsgemäßen metallorganischen Gerüstmaterial kann das Adsorbens beziehungsweise der Adsorber weitere Adsorbentien wie Molekularsiebe oder dergleichen enthalten.

Vorzugsweise ist der Adsorberreaktor Teil eines Adsorbersystems, das mindestens drei Adsorber umfasst, die phasenversetzt arbeiten.

Hierdurch ist es möglich, das Freisetzen des Propans in quasi-kontinuierlicher Fahrweise durchzuführen.

Die Freisetzung des Propans erfolgt vorzugsweise durch Änderung von zumindest einem der physikalischen Parameter ausgewählt aus der Gruppe bestehend aus Druck und Temperatur. Bevorzugt erfolgt zumindest eine Druckänderung.

Das Freisetzen durch Druckänderung kann durch Druckverminderung bis hin zum Anlegen von Vakuum erfolgen. Im Rahmen der vorliegenden Erfindung ist jedoch auch die Verringerung des Partialdruckes von Propan ausreichend, um dieses freizusetzen.

Dies kann beispielsweise durch Verdrängen des Propans mit Inertgas, welches später leicht wieder abgetrennt werden kann, erfolgen.

Das in Kontakt bringen gemäß dem erfindungsgemäßen Verfahren stellt eine Adsorptionsstufe dar, wobei eine Desorptionsstufe zur Gewinnung des Propans erfolgen kann. Erfolgt nun die Adsorption sowie die Desorption unter wechselnder Änderung von Druck und/oder Temperatur, sind dem Fachmann zahlreiche Möglichkeiten einer technischen Realisierung bekannt.

Allen gemeinsam ist, dass wenigstens zwei, vorzugsweise drei, besonders bevorzugt wenigstens vier Adsorber parallel betrieben werden, von denen mindestens zwei, bevorzugt aber alle bezüglich der jeweils anderen Adsorber phasenversetzt arbeiten. Mögliche Variante sind a) eine Druckwechseladsorption ("pressure-swing adsorption", PSA) b) eine Vakuum-Druckwechseladsorption ("vaccuum pressure-swing adsorption", VPSA), c) eine Temperaturwechseladsorption ("temperature-swing adsorption", TSA) oder eine Kombination verschiedener Verfahren. Diese Verfahren sind dem Fachmann prinzipiell bekannt und können in Lehrbüchern wie beispielsweise W. Kast, "Adsorption aus der Gasphase - Ingenieurwissenschaftliche Grundlagen und technische Verfahren", VCH Weinheim, 1988, D. M. Ruthven, S. Farooq, K. S. Knaebel, "Pressure Swing Adsorption", Wiley-VCH, New Y-ork-Chichester-Weinheim-Brisbane-Singapore-Toronto, 1994 oder D. Bathen, M. Breitbach, "Adsorptionstechnik", Springer Verlag Berlin-Heidelberg, 2001, D. Basmadjian, "The Little Adsorption Book", CRC Press Boca Raton, 1996 oder Publikationen wie beispielsweise A. Mersmann, B. Fill, R. Hartmann, S. Maurer, Chem. Eng. Technol. 23/11 (2000) 937 nachgelesen werden. Das Bett eines Adsorbers muss dabei nicht notwendigerweise nur ein einziges Adsorbens enthalten, sondern kann aus mehreren Schichten unterschiedlicher Materialien bestehen. Dies kann beispielsweise genutzt werden, um die Durchbruchsfront der adsorbierten Spezies während der Adsorptionsphase zu schärfen.

Beispielsweise kann eine Druckwechseladsorption für die Propan/Propen-Trennung folgendermaßen ausgestaltet sein: Vier Reaktoren arbeiten parallel in folgenden versetzten Phasen: In Phase 1 wird ein Adsorber durch Zufuhr von Frischgas, Gas aus einem zweiten Adsorber im Adsorptionsmodus bzw. Abgas aus einem zweiten Adsorber, der zeitgleich dekomprimiert wird, auf den Arbeitsdruck (pₘₐₓᵢₘₐₗ) gebracht. In Phase 2 wird das Adsorbens durch weitere Feedzufuhr vollständig mit Propan beladen, bevorzugt bis die gesamte Adsorptionsfront durchgebrochen ist und kein Propan mehr adsorbiert wird. In diesem Fall wird vor Durchbruch der Propanfront bevorzugt ein zweiter Reaktor stromabwärts im Adsorptionsmodus zugeschaltet. In Phase 3 wird der Adsorber mit Propan gespült. Das Spülen kann im Gleich- oder Gegenstrom erfolgen, wobei der Gleichstrom bevorzugt ist. Das Spülen kann bei Adsorptionsdruck erfolgen. Weiterhin wird ein vorheriges Absenken des Adsorberdrucks bevorzugt, insbesondere bevorzugt ist ein ähnlicher Propanpartialdruck in der Adsorptions- (Phase 2) und Spülphase (Phase 3). Das bei dieser Druckabsenkung freiwerdende Gasgemisch kann einem anderen Adsorber während der Phase 1 zum Druckaufbau zugeführt werden. In Phase 4 wird der beladene und gespülte Adsorber dekomprimiert. Das Produkt wird dabei bevorzugt im Gegenstrom abgeführt.

Zusätzlich kann in Phase 4 ein Unterdruck angelegt werden. Diese Ausführungsform ist ein Beispiel für ein VPSA-Verfahren.

Zur Kompensation der Temperatureffekte aufgrund der Adsorptionswärme/Desorptionskälte kann die Zu- oder Abfuhr von Wärme vorteilhaft sein. Der Wärmeintrag kann dabei auf unterschiedliche Arten erfolgen: Konduktiv über innen liegende Wärmetauscher, konvektiv über außen liegende Wärmetauscher oder durch Strahlung, beispielsweise durch Einstrahlung von Mikro- oder Radiowellen. Ebenso kann ein über die Kompensation der Desorptionskälte hinausgehender Wärmeeintrag zur zusätzlichen Erleichterung der Desorption während Phase 4 genutzt werden. Ein derartiges Verfahren stellt eine Kombination aus einer Druckwechsel- und einer Temperaturwechseladsorption dar.

Die Desorption kann auch durch Verdrängung mit einer Hilfskomponente, beispielsweise N₂, CO₂ oder Wasserdampf, erfolgen. Dabei wird ausgenutzt, dass die Hilfskomponente den Partialdruck des Propans in der Gasphase absenkt, während der absolute Druck konstant bleiben kann. Zusätzlich kann eine stärker adsorbierende Hilfskomponente, wie beispielsweise Wasserdampf oder CO₂, auch zu einer Verdrängung von der Oberfläche des Adsorbens führen. In letzterem Fall muss jedoch die Hilfskomponente in einem weiteren Schritt wieder von der Oberfläche des Adsorbens entfernt werden, z. B. durch Anheben der Temperatur.

Die Phasen müssen nicht notwendiger Weise gleich lang dauern, so dass zur Synchronisation auch eine kleinere oder größere Anzahl an Adsorbern eingesetzt werden kann.

Die Adsorption wird im Allgemeinen bei einer Temperatur im Bereich von -20 bis 150 °C, bevorzugt 0 bis 100°C und besonders bevorzugt 10 bis 60°C durchgeführt.

Die Adsorption erfolgt bevorzugt bei einem Druck von im Allgemeinen 2 bis 30 bar, mehr bevorzugt bei 2 bis 5,5 bar. Weiterhin ist bevorzugt 12 bis 25 bar und insbesondere bei 19 bis 21 bar.

Die Desorptionsphase selbst kann sowohl durch Druckerniedrigung als auch durch Wärmeeintrag als auch durch eine Kombination beider Maßnahmen erfolgen. Die Druckerniedrigung erfolgt vorzugsweise auf einen Druck von unter 2,5 bar, insbesondere unter 2 bar.

Die angegebenen Druckwerte stellen Absolutwerte dar.

Die Adsorption/Desorption kann als Festbett-, Wirbelschicht- oder Wanderbettverfahren ausgestaltet sein. Geeignete Apparate sind zum Beispiel Festbettreaktoren, Rotationsadsorber oder Jalousienfilter. Eine ausführliche Beschreibung von möglichen Apparaten findet sich in: Werner Kast, "Adsorption aus der Gasphase", VCH (Weinheim); H. Brauer, "Die Adsorptionstechnik ein Gebiet mit Zukunft", Chem.-Ing. Tech 57 (1985) 8, 650-653; Dieter Bathen, Marc Breitbach "Adsorptionstechnik", VDI-Buch, 2001.

Zur Desorption der an dem Adsorbens adsorbierten Gase wird dieses erhitzt und/oder auf einen niedrigeren Druck entspannt.

Vorzugsweise kann gemäß dem erfindungsgemäßen Verfahren zur technischen Gewinnung von Propen das freigesetzte Propen mit einer Reinheit von über 80 Vol-% in Bezug auf die Summe der Volumenanteile an Propan und Propen erhalten werden. Mehr bevorzugt beträgt die Reinheit mehr als 90 Vol-%, mehr bevorzugt mindestens 95 Vol-%, insbesondere mindestens 98,5 Vol-%.

Ein weiterer Gegenstand der vorliegenden Erfindung ist die Verwendung eines porösen metallorganischen Gerüstmaterials enthaltend mindestens eine an mindestens ein Metallion koordinativ gebundene mindestens zweizähnige organische Verbindung zur technischen Gewinnung von Propen aus einem zumindest Propen und Propan enthaltenden Gasstrom durch Propanabreicherung des Gasstromes, wobei die mindestens zweizähnige organische Verbindung ein Imidazolat ist, das unsubstituiert ist oder einen oder mehrere Substituenten unabhängig voneinander ausgewählt aus der Gruppe bestehend aus Halogen, C₁₋₆ Alkyl, Phenyl, NH₂, NH(C₁₋₆ Alkyl), N(C₁₋₆ Alkyl)₂, OH, O-Phenyl und O-C₁₋₆ Alkyl aufweist.

### Beispiele

### Beispiel 1 Elektrochemische Herstellung des erfindungsgemäßen Adsorbens Zn(II)-2-Methylimidazolat (Zn(MIM)₂)

Der Elektrolyt, bestehend aus 76,1 g 2-Methylimidazol, 85,8 g Methyltributylammoniummethylsulfat (MTBS), 1810 g Methanol und 750,2 g Wasser wird in einen Zellkreislauf eingefüllt. Eine Leitfähigkeit von 4,8 mS/cm wird gemessen.

Der Zellkreislauf besteht aus einer Rohrzelle, einem Glaskühler und einer Kreislaufpumpe. Die Pumpe förderte den Elektrolyten bzw. die entstehende Suspension mit ca. 600 l/h im Kreis.

Die Rohrzelle besteht aus einem Edelstahlrohr (Länge: 55 cm, Innendurchmesser: 5 cm) als Kathode und einem Zinkstab als Anode (Länge: 55 cm, Durchmesser: 1,94 cm, Oberfläche: 3,41 cm²). Die Anordnung in der Elektrolysezelle stellt durch verschiedene luftdichte Dichtungen und Verschraubungen sicher, dass die Elektroden konzentrisch angeordnet sind und garantieren einen ringsum homogenen Spalt zwischen Kathode und Anode, durch den der auf 29°C thermostatisierte Elektrolyt gepumpt wird.

Bei einer Stromstärke von 5,1 A und einer Zellspannung von 4,6 bis 5 V wird bis zu einem Stromeinsatz von 1 Faraday pro Mol 2-Methylimidazol für 4,8 Stunden gefahren (24,6 Ah). Während des Versuches wird die Zelle mit einem Inertgasstrom gespült, um entstehenden Wasserstoff zu entfernen und eine Knallgasbildung auszuschließen.

Nach beendeter Elektrolyse wird der Elektrolyt filtriert und mit 300 g Methanol gewaschen. Das Gewicht der Zinkanode verringert sich um 29,0 g. Das kristalline Produkt wird bei 80°C bei 1 mbar getrocknet und es werden 100,9 g Zn(MIM)₂ erhalten (Ausbeute 98%). Die Oberfläche wird bestimmt nach Langmuir gemäß DIN 66135 und liegt bei 1718 m²/g.

### Beispiel 2 Abtrennung von Propan aus einem Propan/Propen-haltigen Gemisch

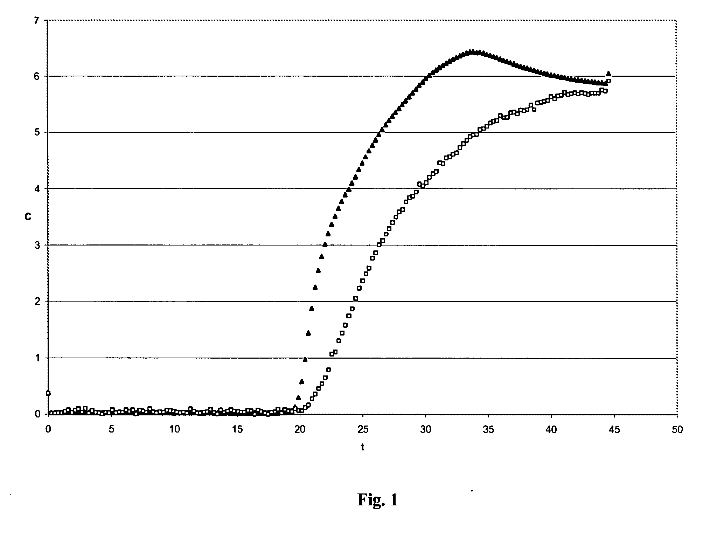

Das Material aus Beispiel 1 wird unter Zusatz von 3 Gew.-% Graphit zu 1,9 x 1,9 mm Tabletten verformt (Oberfläche 1266 m²/g) und zu Splitt (0,5 - 1 mm) gebrochen. Ein Adsorberreaktor (Durchmesser ca. 2.5 cm) wird mit 50 g des Materials befüllt. Vor dem Versuch wird die Schüttung mehr als 16 h mit trockenem Stickstoff bei 130°C gespült. Anschließend wird bei 25°C mit reinem He ein Druck von 7.5 bar aufgebaut und unter diesen Bedingungen ein Gemisch von 5% Propan und 5% Propen in Helium auf den Reaktor gestellt. Die Zusammensetzung des austretenden Gases wird online mittels Infrarot-Spektrometrie (IR) überwacht. Der Verlauf des IR-Signals für das austretende Gas (Konzentration C in %) ist als Funktion der Zeit t in min. in Fig. 1 dargestellt. Zunächst adsorbieren beide Komponenten auf der Oberfläche, so dass keine der Komponenten in der IR-Zelle nachgewiesen wird. Nach einiger Zeit bricht als erstes die Propenkomponente (schwarze Symbole) durch, was anhand des Signal-Anstiegs in der Infrarot-Zelle nachgewiesen wird. Das Propan wird jedoch noch für einige Zeit weiterhin adsorbiert und bricht erst zu einem späteren Zeitpunkt durch (weiße Symbole).

### Vergleichsbeispiel 3 Trennung eines Propan/Propen-Gemisches an einem 13X-Molekularsieb

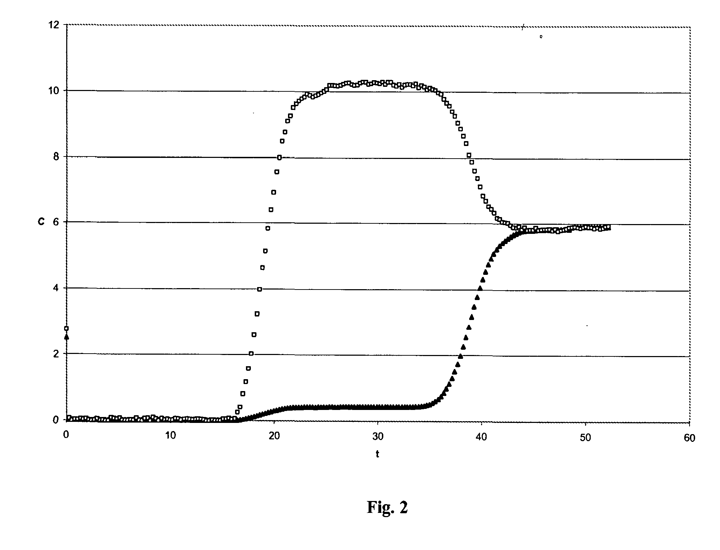

Als Vergleich wird der Reaktor mit 50 g eines "Molekularsieb 5A" (Fa. Carl Roth GmbH + Co. KG, Schoemperlenstr. 3-5, Karlsruhe, Deutschland) befüllt. Das Molekularsieb wird zuvor in einem Vakuumtrockenschrank 10 h bei 180°C vorgetrocknet. Der Versuch wird analog zu Beispiel 2 durchgeführt. Das Ergebnis ist in Fig. 2 dargestellt (Propen: schwarze Symbole, Propan: weiße Symbole), wobei C und t die gleiche Bedeutung wie in Fig. 1 haben. Als erstes bricht in diesem Fall Propan durch, während Propen weiterhin adsorbiert wird und erst nach vollständiger Sättigung des Sorbens bricht Propen zu einem späteren Zeitpunkt durch.

## Patentansprüche

1. Verfahren zur technischen Gewinnung von Propen aus einem zumindest Propen und Propan enthaltenden Gasstrom, den Schritt enthaltend
- In Kontakt bringen des Gasstromes mit einem Adsorbens enthaltend ein poröses metallorganisches Gerüstmaterial enthaltend mindestens eine an mindestens ein Metallion koordinativ gebundene mindestens zweizähnige organische Verbindung, wobei das Adsorbens mit Propan beladen wird und der Gasstrom dadurch einen erhöhten Propenanteil aufweist, wobei die mindestens zweizähnige organische Verbindung ein Imidazolat ist, das unsubstituiert ist oder einen oder mehrere Substituenten unabhängig voneinander ausgewählt aus der Gruppe bestehend aus Halogen, C₁₋₆ Alkyl, Phenyl, NH₂, NH(C₁₋₆ Alkyl), N(C₁₋₆ Alkyl)₂, OH, O-Phenyl und O-C₁₋₆ Alkyl aufweist.

2. Verfahren nach Anspruch 1, **dadurch gekennzeichnet, dass** der Gasstrom 20 Vol-% bis 80 Vol-% Propen bezogen auf die Summe der Volumenanteile an Propen und Propan enthält.

3. Verfahren nach Anspruch 1 oder 2, **dadurch gekennzeichnet, dass** der Gastrom ein gegebenenfalls gereinigter Produktstrom aus der Propenherstellung ist.

4. Verfahren nach Anspruch 3, **dadurch gekennzeichnet, dass** der gegebenenfalls gereinigte Produktstrom aus einem Crack-Verfahren, einer Propandehydrierung, einer Olefinumwandlung oder einer Methanol/Dimethylether-Umwandlung zur Propenherstellung oder einer Mischung von Strömen zweier oder mehrer dieser Propenherstellungen stammt.

5. Verfahren Anspruch 4, **dadurch gekennzeichnet, dass** der gegebenenfalls gereinigte Produktstrom aus einer Propandehydrierung zur Propenherstellung stammt.

6. Verfahren nach einem der Ansprüche 1 bis 5, **dadurch gekennzeichnet, dass** der Gasstrom einen Propengehalt nach dem in Kontakt bringen von über 80 Vol-% in Bezug auf die Summe der Volumenanteile Propen und Propan aufweist.

7. Verfahren nach einem der Ansprüche 1 bis 6, **dadurch gekennzeichnet, dass** mindestens eine der folgenden Bedingungen erfüllt ist:
(a) das in Kontakt bringen erfolgt bei einer Temperatur im Bereich von -20 °C bis 150 °C;
(b) das in Kontakt bringen erfolgt bei einem Druck im Bereich von 2 bar (absolut) bis 30 bar (absolut).

8. Verfahren nach einem der Ansprüche 1 bis 7, **dadurch gekennzeichnet, dass** das mindestens eine Metallion ausgewählt ist aus der Gruppe der Metalle bestehend aus Kupfer, Eisen, Aluminium, Zink, Magnesium, Zirkonium, Titan, Vanadium, Molybdän, Wolfram, Indium, Calcium, Strontium, Kobalt, Nickel, Platin, Rhodium, Ruthenium, Palladium, Scandium, Yttrium, ein Lanthanid, Mangan und Rhenium.

9. Verfahren nach einem der Ansprüche 1 bis 8, **dadurch gekennzeichnet, dass** das Imidazolat einen oder mehrere C₁₋₆ Alkyl-Substituenten aufweist.

10. Verwendung eines porösen metallorganischen Gerüstmaterials enthaltend mindestens eine an mindestens ein Metallion koordinativ gebundene mindestens zweizähnige organische Verbindung zur technischen Gewinnung von Propen aus einem zumindest Propen und Propan enthaltenden Gasstrom durch Propanabreicherung des Gasstromes, wobei die mindestens zweizähnige organische Verbindung ein Imidazolat ist, das unsubstituiert ist oder einen oder mehrere Substituenten unabhängig voneinander ausgewählt aus der Gruppe bestehend aus Halogen, C₁₋₆ Alkyl, Phenyl, NH₂, NH(C₁₋₆ Alkyl), N(C₁₋₆ Alkyl)₂, OH, O-Phenyl und O-C₁₋₆ Alkyl aufweist.

## Claims

1. A process for the industrial isolation of propene from a gas stream comprising at least propene and propane, which comprises the step
- contacting of the gas stream with an adsorbent comprising a porous metal organic framework comprising at least one at least bidentate organic compound coordinated to at least one metal ion, with the adsorbent becoming laden with propane and the gas stream therefore having an increased proportion of propene, wherein the at least bidentate organic compound is an imidazolate which is unsubstituted or has one or more substituents selected independently from the group consisting of halogen, C₁₋₆-alkyl, phenyl, NH₂, NH(C₁₋₆-alkyl), N(C₁₋₆-alkyl)₂, OH, O-phenyl and O-C₁₋₆-alkyl.

2. The process according to claim 1, wherein the gas stream comprises from 20% by volume to 80% by volume of propene based on the sum of the proportions by volume of propene and propane.

3. The process according to claim 1 or 2, wherein the gas stream is an optionally purified product stream from the preparation of propene.

4. The process according to claim 3, wherein the optionally purified product stream originates from a cracking process, a dehydrogenation of propane, an olefin transformation or a methanol/dimethyl ether transformation for the preparation of propene or a mixture of streams from two or more of these preparations of propene.

5. The process according to claim 4, wherein the optionally purified product stream originates from a dehydrogenation of propane to prepare propene.

6. The process according to any of claims 1 to 5, wherein the gas stream has a propene content after contacting of over 80% by volume based on the sum of the proportions by volume of propene and propane.

7. The process according to any of claims 1 to 6, wherein at least one of the following conditions is fulfilled:
(a)the contacting is carried out at a temperature in the range from -20°C to 150°C;
(b)the contacting is carried out at a pressure in the range from 2 bar (absolute) to 30 bar (absolute).

8. The process according to any of claims 1 to 7, wherein the at least one metal ion is selected from the group of metals consisting of copper, iron, aluminum, zinc, magnesium, zirconium, titanium, vanadium, molybdenum, tungsten, indium, calcium, strontium, cobalt, nickel, platinum, rhodium, ruthenium, palladium, scandium, yttrium, a lanthanide, manganese and rhenium.

9. The process according to any of claims 1 to 8, wherein the imidazolate has one or more C₁₋₆-alkyl substituents.

10. The use of a porous metal organic framework comprising at least one at least bidentate organic compound coordinated to at least one metal ion for the industrial isolation of propene from a gas stream comprising at least propene and propane by at least partial removal of propane from the gas stream, wherein the at least bidentate organic compound is an imidazolate which is unsubstituted or has one or more substituents selected independently from the group consisting of halogen, C₁₋₆-alkyl, phenyl, NH₂, NH(C₁₋₆-alkyl), N(C₁₋₆-alkyl) ₂, OH, 0-phenyl and O-C₁₋₆-alkyl.

## Revendications

1. Procédé pour l'obtention industrielle de propène à partir d'un courant gazeux contenant au moins du propène et du propane, comportant l'étape
- mise en contact du courant gazeux avec un adsorbant comportant une matière organométallique poreuse de support contenant au moins un composé organique au moins bidenté lié par coordination à au moins un ion métallique, de sorte que l'adsorbant se charge de propane et le courant gazeux présente ainsi une teneur accrue en propène, le composé organique au moins bidenté étant un imidazolate qui est non substitué ou comporte un ou plusieurs substituants choisis indépendamment les uns des autres dans l'ensemble constitué par des atomes d'halogène, des groupes alkyle en C₁-C₆, phényle, NH₂, NH(alkyle en C₁-C₆), N(alkyle en C₁-C₆)₂, OH, O-phényle et O-alkyle en C₁-C₆.

2. Procédé selon la revendication 1, **caractérisé en ce que** le courant gazeux contient 20 % en volume à 80 % en volume de propène, par rapport à la somme des proportions en volume de propène et propane.

3. Procédé selon la revendication 1 ou 2, **caractérisé en ce que** le courant gazeux est un courant de produit éventuellement purifié provenant de la production du propène.

4. Procédé selon la revendication 3, **caractérisé en ce que** le courant de produit éventuellement purifié provient d'un processus de craquage, d'une déshydrogénation du propane, d'une conversion d'oléfine ou d'une conversion méthanol/éther diméthylique pour la production de propène ou d'un mélange de courants de deux ou plus de deux de ces productions de propène.

5. Procédé selon la revendication 4, **caractérisé en ce que** le courant de produit éventuellement purifié provient d'une déshydrogénation du propane destinée à la production du propène.

6. Procédé selon l'une quelconque des revendications 1 à 5, **caractérisé en ce qu'**après la mise en contact le courant gazeux présente une teneur en propène de plus de 80 % en volume par rapport à la somme des proportions en volume de propène et propane.

7. Procédé selon l'une quelconque des revendications 1 à 6, **caractérisé en ce qu'**au moins l'une des conditions suivantes est remplie :
(a) la mise en contact s'effectue à une température dans la plage de -20 °C à 150 °C ;
(b) la mise en contact s'effectue sous une pression de 2 bars (absolue) à 30 bars (absolue).

8. Procédé selon l'une quelconque des revendications 1 à 7, **caractérisé en ce qu'**au moins un ion métallique est choisi dans le groupe des métaux consistant en le cuivre, le fer, l'aluminium, le zinc, le magnésium, le zirconium, le titane, le vanadium, le molybdène, le tungstène, l'indium, le calcium, le strontium, le cobalt, le nickel, le platine, le rhodium, le ruthénium, le palladium, le scandium, l'yttrium, un lanthanide, le manganèse et le rhénium.

9. Procédé selon l'une quelconque des revendications 1 à 8, **caractérisé en ce que** l'imidazolate comporte un ou plusieurs substituants alkyle en C₁-C₆.

10. Utilisation d'une matière organométallique poreuse de support contenant au moins un composé organique au moins bidenté lié par coordination à au moins un ion métallique, pour l'obtention industrielle de propène à partir d'un courant gazeux contenant au moins du propène et du propane, par abaissement de la teneur en propane du courant gazeux, ledit composé organique au moins bidenté étant un imidazolate qui est non substitué ou comporte un ou plusieurs substituants choisis indépendamment les uns des autres dans l'ensemble constitué par des atomes d'halogène, des groupes alkyle en C₁-C₆, phényle, NH₂, NH(alkyle en C₁-C₆), N(alkyle en C₁-C₆)₂, OH, O-phényle et O-alkyle en C₁-C₆.
